# EUROPEAN PATENT APPLICATION

(11) **EP 2 325 159 A1**
(43) Date of publication of application: **25.05.2011**
(21) Application number: 09176947.1
(22) Date of filing: 24.11.2009
(51) Int. Cl.: C07C 59/11, C07C 211/42, C07C 309/23, A61K 31/135

(54) **Novel salts of rasagiline**

(71) Applicant: LEK Pharmaceuticals d.d., 1526 Ljubljana (SI)
(72) Inventor: The designation of the inventor has not yet been filed

(57) **Abstract**

The present invention relates to novel pharmaceutically acceptable salts of rasagiline, to processes for their preparation and to pharmaceutical compositions containing them.

## Description

### Field of the Invention

The present invention relates to novel pharmaceutically acceptable salts of rasagiline, to processes for their preparation and to pharmaceutical compositions containing them.

### Background of the Invention

The R(+) enantiomer of N-propargyl-1-aminoindane, also named rasagiline, is a potent, irreversible monoamine-oxidase type B inhibitor (MAO-B), which is used in particular to treat Parkinson's disease and Alzheimer type dementia. Further indications have been disclosed in EP436492B1, WO95/11016 and WO96/37199. The compound was initially disclosed in US patent 3201470 and in DE1443599. Rasagiline as an individual compound was described in EP436492B1, together with its pharmaceutically acceptable salts, in particular the hydrochloride, tartrate and mesylate salts of rasagiline. Furthermore, the same document discloses also ways, in which the R(+) enantiomer of N-propargyl-1-aminoindane can be obtained. Among others, chiral resolution utilizing chiral acids such as tartaric, malic, mandelic acid, or N-acetyl derivatives of aminoacids is mentioned, and an example of said method using tartaric acid as a resolving agent is described.

Other pharmaceutically acceptable salts of rasagiline, namely maleate, fumarate, hydrobromide, esylate, p-toluensulfate, benzoate, acetate, phosphate and sulfate, have been disclosed in WO95/11016.

International patent application WO08/019871 relates to rasagiline salts that are stable during longer storage, and to tablets compressed there from. Oxalate and edisilat salts are particularly preferred.

The subject matter of WO08/76315 is a tannate salt of rasagiline, pharmaceutical compositions comprising this salt, as well as the process of its preparation. Rasagiline tannate has low water solubility and may be used for transdermal and delayed or extended release oral dosage forms.

Salts often improve physical and biological characteristics of mother compounds without modifying primary pharmacological activity, based on mechanism of action. Thus there is a continuous need to obtain new salts of rasagiline having suitable physical and/or chemical properties. The present invention satisfies this need by providing new alternative salts of rasagiline with suitable water solubility and polymorph stability. In addition, the resolving agents used for obtaining said salts provide an enhanced means for chiral resolution of rasagiline.

### Summary of the invention

The present invention provides the following items including main aspects and preferred embodiments, which respectively alone and in combination particularly contribute to solving the above object and eventually provide additional advantages:
(1) Rasagiline salt with a pharmaceutically acceptable acid, the pharmaceutically acceptable acid being selected from the group consisting of L-mandelic acid, (+)-camphor-10-sulfonic acid, D-mandelic acid, (-)-camphor-10-sulfonic acid, L-malic acid, D-malic acid, orotic acid, 1-hydroxy-2-naphthoic acid, benzoic acid, fumaric acid, crotonic acid, glutaric acid, citric acid, L-lactic acid, racemic lactic acid, succinic acid, and trans-cinnamic acid, and hydrates and solvates of said salt.
(2) Rasagiline salt according to item 1, wherein the pharmaceutically acceptable acid is selected from the group consisting of L-mandelic acid and (+)-camphor-10-sulfonic acid, and hydrates and solvates of said salt.
(3) Rasagiline salt according to item 2, which is rasagiline L-mandelate, or a hydrate or solvate thereof, optionally being in crystalline form or in amorphous form.
(4) Rasagiline L-mandelate according to item 3 being in crystalline form and having a powder X-ray diffraction pattern comprising the following characteristic reflection angles 20: 5.4±0.2 °, 9.7±0.2 °, 10.8±0.2 °., 17.2±0.2 °, 19.4±0.2 °, 20.5±0.2 °, 21.9±0.2 °, 27.0±0.2 ° and 29.6±0.2 °.
(5) Rasagiline L-mandelate according to any one of items 3 or 4 having a melting point falling in a range of 106 to 118°C, preferably 108 to 114°C.
(6) Rasagiline salt according to item 2, which is rasagiline (+)-camphor-10-sulfonate, or a hydrate or solvate thereof, optionally being in crystalline form or in amorphous form.
(7) Rasagiline (+)-camphor-10-sulfonate according to item 6 being in crystalline form and having a powder X-ray diffraction pattern comprising the following characteristic reflection angles 20: 7.6±0.2 °, 13.1 ±0.2 °, 18.1 ±0.2 °, 19.0±0.2 °, 21.6±0.2 °, 23.1 ±0.2 °, 25.7±0.2 ° and 29.5±0.2 °.
(8) Rasagiline (+)-camphor-10-sulfonate according to any one of items 6 or 7 having a melting point falling in a range of 162 to 175°C, preferably 167 to 173 °C.
(9) A process for the preparation of a rasagiline salt according to any one of items 1-8 comprising the following steps:
   a) providing a mixture comprising racemic N-propargyl-1-aminoindane or rasagiline base, and an organic acid selected from the group consisting of L-mandelic acid, (+)-camphor-10-sulfonic acid, D-mandelic acid, (-)-camphor-10-sulfonic acid, L-malic acid, D-malic acid, orotic acid, 1-hydroxy-2-naphthoic acid, benzoic acid, fumaric acid, crotonic acid, glutaric acid, citric acid, L-lactic acid, racemic lactic acid, succinic acid, and trans-cinnamic acid;
   b) isolating the obtained rasagiline salt.
(10) The process according to item 9, wherein said organic acid is selected from the group consisting of L-mandelic acid and (+)-camphor-10-sulfonic acid.
(11) The process according to item 9 or 10, wherein said mixture is provided in 2-propanol as solvent.
(12) A pharmaceutical composition comprising a rasagiline salt according to any one of items 1 to 8.
(13) The pharmaceutical composition according to item 12, wherein said rasagiline salt is rasagiline L-mandelate.
(14) The pharmaceutical composition according to item 12, wherein said rasagiline salt is rasagiline (+)-camphor-10-sulfonate.
(15) A pharmaceutical composition according to any one of items 12 to 14 for use in therapeutic treatment of Parkinson's disease, memory disorders, dementia of the Alzheimer type, depression, hyperactive syndrome, a neurotoxic injury, brain ischemia or stroke, head trauma injury or spinal trauma injury, neurotrauma, schizophrenia, attention deficit disorder; multiple sclerosis, nerve damage, an affective illness, symptoms of withdrawal from an addictive substance, amyotrophic lateral sclerosis, multiple system atrophy, restless leg syndrome, glaucoma, hairloss.

Due to imperfect physical-chemical properties of rasagiline base, e.g. low solubility in water, the commercialized product (Azilect^{®}) comprises a mesylate salt of rasagiline. The mesylate salt and the majority of other known salts of rasagiline are hygroscopic. This property may cause the formation of agglutinates during the synthesis and as a consequence problems in the process of preparing final dosage forms, for example tablets, may be encountered.

It must be emphasized that the existence and properties of individual salts are inherently unpredictable. Hence although numerous acids are available to try as alternatives, the skilled person cannot predict which, if any, is likely to provide a salt of rasagiline having physical and/or chemical properties suitable to be included into a pharmaceutical composition, and if so, which one can provide appropriate dosage or administration forms.

Chiral resolution of racemic amines with optically active acids represents a long standing practice in synthetic chemistry, and has also been utilized for optical resolution of N-propargyl-1-aminoindane to yield the desired R(+) enantiomer. However, finding an acid suitable for chiral resolution, which would give a rasagiline salt of suitable enantiomeric purity and would in addition possess good water solubility, currently still represents an unmet need.

The inventor of present invention has now recognized that by using L-mandelic acid and (+)-camphor-10-sulfonic acid as chiral agents in the process of chiral resolution of N-propargyl-1-aminoindane racemic base, two new salts of rasagiline, namely rasagiline L-mandelate and rasagiline (+)-camphor-10-sulfonate are obtained, which surprisingly possess markedly enhanced physical-chemical properties over other known salts prepared by chiral resolution, e.g. better water solubility over tartrate salt. Moreover, these salts do not form agglutinates and are additionally easy to handle and process, and are therefore suitable for the manufacture of various pharmaceutical dosage forms. Excellent solubility in water while still remaining thermally stable ensures valuable and useful performance for the resulting pharmaceutical composition.

### Brief Description of the Figures

Fig. 1 shows a comparison of solubility curves for L-mandelate, (+)-camphor-10-sulfonate and L-tartrate salts of rasagiline in water;
Fig. 2 is a characteristic X-Ray diffraction pattern of crystalline rasagiline L-mandelate of present invention.

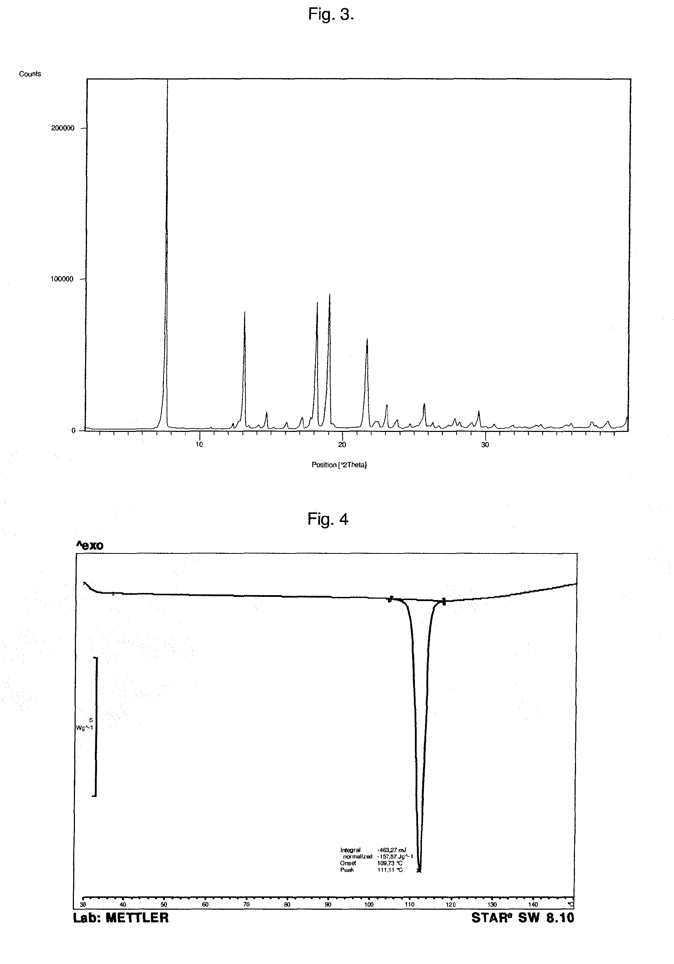

Fig. 3 is a characteristic X-Ray diffraction pattern of crystalline rasagiline (+)-camphor-10-sulfonate of present invention.
Fig. 4 shows DSC thermogram of rasagiline L-mandelate

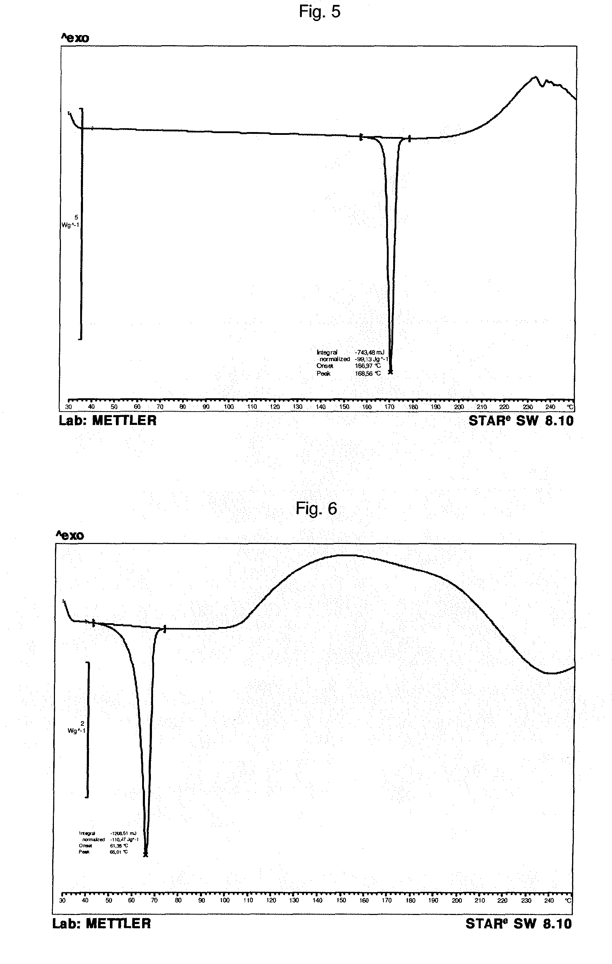

Fig. 5 shows DSC thermogram of rasagiline (+)-camphor-10-sulfonate.
Fig. 6 shows DSC thermogram of rasagilin benzoate.

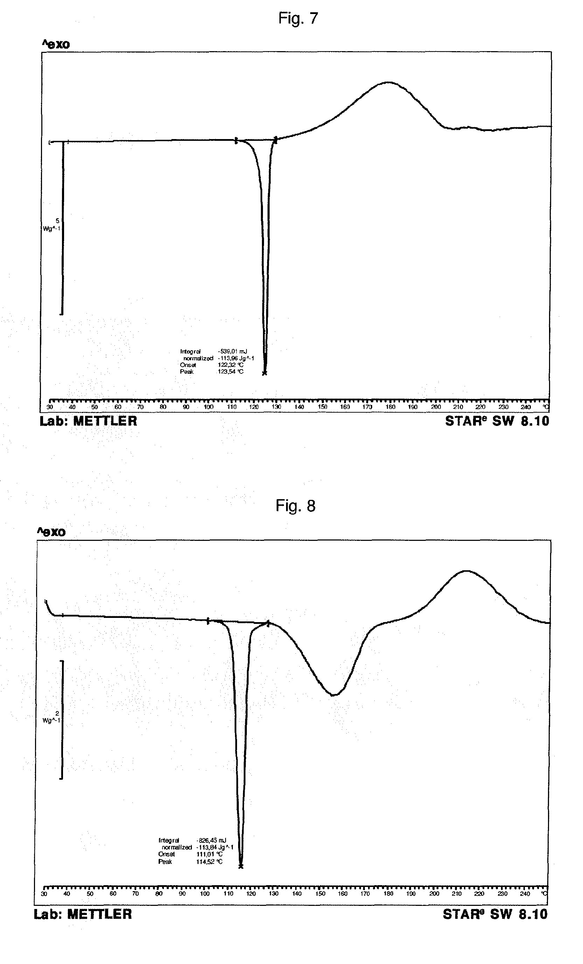

Fig. 7 shows DSC thermogram of rasagiline fumarate.
Fig. 8 shows DSC thermogram of rasagiline 1-hydroxy-2-naphthoate.

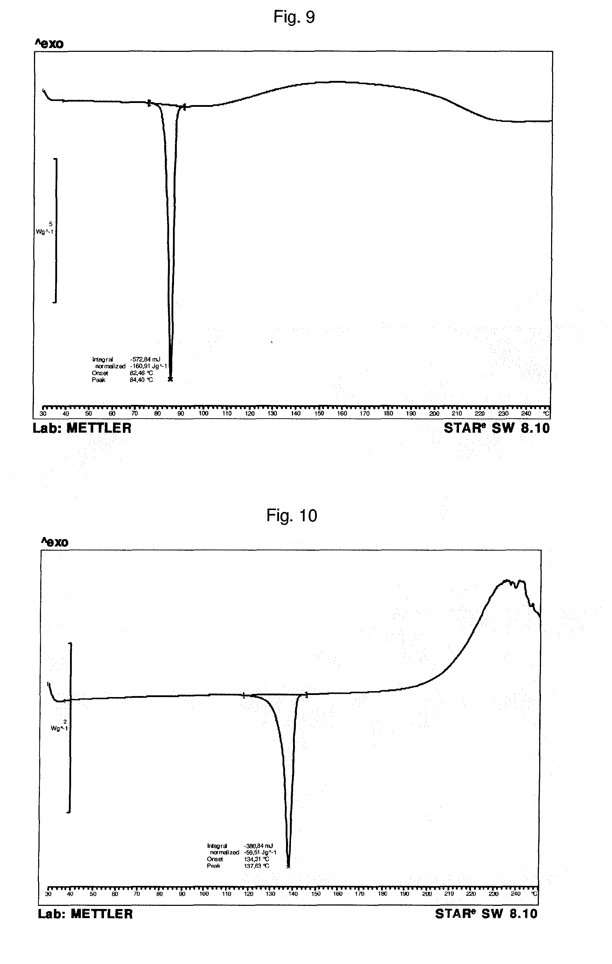

Fig. 9 shows DSC thermogram of rasagiline cinnamate.
Fig. 10 shows DSC thermogram of rasagiline (-)-camphor-10-sulfonate.

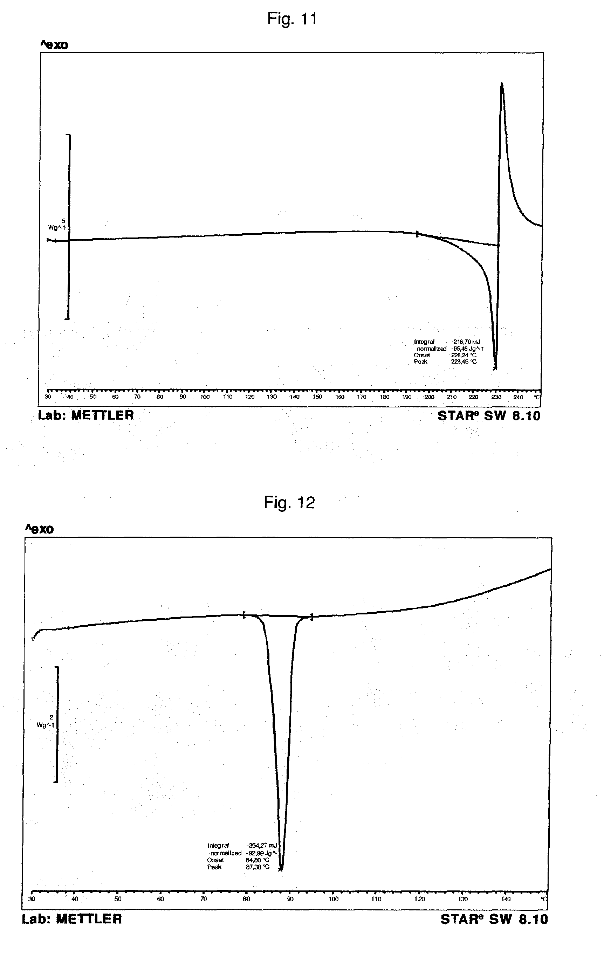

Fig. 11 shows DSC thermogram of rasagiline orotate.
Fig. 12 shows DSC thermogram of rasagiline D-mandelate.

### Detailed description of the Invention

Rasagiline is a chiral compound having an asymmetric carbon atom in an absolute R(+) configuration. Although the S(-) enantiomer of N-propargyl-1-aminoindane still exerts some neuroprotective properties, the potency of R(+) enantiomer against the MAO-B enzyme is approximately 1000-fold higher. Therefore, it is essential to separate the isomers; only R(+) enantiomer, that is rasagiline, should be included in the pharmaceutical composition.

In the following, the present invention will be described in more detail by preferred embodiments and examples while referring to the attached drawings, noting, however, that these embodiments, examples and drawings are presented for illustrative purposes only and shall not limit the invention in any way.

In one aspect the present invention relates to novel salts of rasagiline with a pharmaceutically acceptable acid selected from the group consisting of L-mandelic acid, (+)-camphor-10-sulfonic acid, D-mandelic acid, (-)-camphor-10-sulfonic acid, L-malic acid, D-malic acid, orotic acid, 1-hydroxy-2-naphthoic acid, benzoic acid, fumaric acid, crotonic acid, glutaric acid, citric acid, L-lactic acid, racemic lactic acid, succinic acid, and trans-cinnamic acid.

In one aspect the present invention relates to hydrates and solvates of salts of rasagiline with a pharmaceutically acceptable acid selected from the group consisting of L-mandelic acid, (+)-camphor-10-sulfonic acid, D-mandelic acid, (-)-camphor-10-sulfonic acid, L-malic acid, D-malic acid, orotic acid, 1-hydroxy-2-naphthoic acid, benzoic acid, fumaric acid, crotonic acid, glutaric acid, citric acid, L-lactic acid, racemic lactic acid, succinic acid, and trans-cinnamic acid.

In another aspect the present invention relates to rasagiline L-mandelate or hydrate or solvate thereof.

In another aspect the present invention relates to crystalline rasagiline L-mandelate.

In another aspect the present invention relates to rasagiline L-mandelate in amorphous form.

In another aspect the present invention relates to rasagiline (+)-camphor-10-sulfonate or hydrate or solvate thereof.

In another aspect the present invention relates to crystalline rasagiline (+)-camphor-10-sulfonate.

In another aspect the present invention relates to rasagiline (+)-camphor-10-sulfonate in amorphous form.

In another aspect the present invention relates to rasagiline benzoate or hydrate or solvate thereof.

In another aspect the present invention relates to crystalline rasagiline benzoate.

In another aspect the present invention relates to rasagiline benzoate in amorphous form.

In another aspect the present invention relates to rasagiline fumarate or hydrate or solvate thereof.

In another aspect the present invention relates to crystalline rasagiline fumarate.

In another aspect the present invention relates to rasagiline fumarate in amorphous form.

In another aspect the present invention relates to rasagiline 1-hydroxy-2-naphthoate or hydrate or solvate thereof.

In another aspect the present invention relates to crystalline rasagiline 1-hydroxy-2-naphthoate.

In another aspect the present invention relates to rasagiline 1-hydroxy-2-naphthoate in amorphous form.

In another aspect the present invention relates to rasagiline cinnamate or hydrate or solvate thereof.

In another aspect the present invention relates to crystalline rasagiline cinnamate.

In another aspect the present invention relates to rasagiline cinnamate in amorphous form.

In another aspect the present invention relates to rasagiline (-)-camphor-10-sulfonate or hydrate or solvate thereof.

In another aspect the present invention relates to crystalline rasagiline (-)-camphor-10-sulfonate.

In another aspect the present invention relates to rasagiline (-)-camphor-10-sulfonate in amorphous form.

In another aspect the present invention relates to rasagiline orotate or hydrate or solvate thereof.

In another aspect the present invention relates to crystalline rasagiline orotate.

In another aspect the present invention relates to rasagiline orotate in amorphous form.

In another aspect the present invention relates to rasagiline D-mandelate or hydrate or solvate thereof.

In another aspect the present invention relates to crystalline rasagiline D-mandelate.

In another aspect the present invention relates to rasagiline D-mandelate in amorphous form.

In another aspect the present invention relates to rasagiline L-malate or hydrate or solvate thereof.

In another aspect the present invention relates to crystalline rasagiline L-malate.

In another aspect the present invention relates to rasagiline L-malate in amorphous form.

In another aspect the present invention relates to rasagiline D-malate or hydrate or solvate thereof.

In another aspect the present invention relates to crystalline rasagiline D-malate.

In another aspect the present invention relates to rasagiline D-malate in amorphous form.

In another aspect the present invention relates to rasagiline crotonate or hydrate or solvate thereof.

In another aspect the present invention relates to crystalline rasagiline crotonate.

In another aspect the present invention relates to rasagiline crotonate in amorphous form.

In another aspect the present invention relates to rasagiline glutarate or hydrate or solvate thereof.

In another aspect the present invention relates to crystalline rasagiline glutarate.

In another aspect the present invention relates to rasagiline glutarate in amorphous form.

In another aspect the present invention relates to rasagiline citrate or hydrate or solvate thereof.

In another aspect the present invention relates to crystalline rasagiline citrate.

In another aspect the present invention relates to rasagiline citrate in amorphous form.

In another aspect the present invention relates to rasagiline L-lactate or hydrate or solvate thereof.

In another aspect the present invention relates to crystalline rasagiline L-lactate.

In another aspect the present invention relates to rasagiline L-lactate in amorphous form.

In another aspect the present invention relates to rasagiline succinate or hydrate or solvate thereof.

In another aspect the present invention relates to crystalline rasagiline succinate.

In another aspect the present invention relates to rasagiline succinate in amorphous form.

Preferred rasagiline salts of the present invention are rasagiline L-mandelate and rasagiline (+)-camphor-10-sulfonate. Particularly preferred is the L-mandelate salt since formation of toxic esters cannot occur, as is expected to happen in case of sulfonate salts (e.g. mesylate and ethanedisulfonate).

In yet another aspect the present invention relates to a process of preparing salts of rasagiline with pharmaceutically acceptable acids selected from the group of L-mandelic acid, (+)-camphor-10-sulfonic acid, D-mandelic acid, (-)-camphor-10-sulfonic acid, L-malic acid, D-malic acid, orotic acid, 1-hydroxy-2-naphthoic acid, benzoic acid, fumaric acid, crotonic acid, glutaric acid, citric acid, L-lactic acid, racemic lactic acid, succinic acid, and trans-cinnamic acid by providing a mixture of racemic N-propargyl-1-aminoindane or rasagiline base and pharmaceutically acceptable acid selected from the group of L-mandelic acid, (+)-camphor-10-sulfonic acid, D-mandelic acid, (-)-camphor-10-sulfonic acid, L-malic acid, D-malic acid, orotic acid, 1-hydroxy-2-naphthoic acid, benzoic acid, fumaric acid, crotonic acid, glutaric acid, citric acid, L-lactic acid, racemic lactic acid, succinic acid, and trans-cinnamic acid, respectively, in a suitable solvent system, comprised of a single solvent or a mixture of solvents, and isolating the obtained rasagiline salt by precipitation, filtration of the solid salt, evaporation, spray drying or other conventional techniques known in the art.

Suitable solvents are solvents selected from alcohols, ketones, nitriles, and esters or mixtures thereof, preferably selected from acetone, C₁-C₄ alcohols, acetonitrile, ethyl acetate. Most preferably 2-propanol is used.

Pharmaceutically acceptable acid in natural state or in solution can be added to the solution of racemic N-propargyl-1-aminoindane or rasagiline base.

Pharmaceutically acceptable acid is preferably added in an equimolar ratio to racemic N-propargyl-1-aminoindane or rasagiline base or an excess of the acid is used.

The temperature of solvent system comprising a mixture of racemic N-propargyl-1-aminoindane or rasagiline base and pharmaceutically acceptable acid is from ambient temperature to the boiling point of the solvent system. After the racemic N-propargyl-1-aminoindane or the rasagiline base and the selected organic acid have been dissolved in a liquid medium the salt is formed. A suitable time for formation of the salt is at least 1 hour, preferably at least 3 hours.

Rasagiline salt can be isolated or recovered from the reaction solution by precipitation. The precipitation can be spontaneous depending on solvent system. Alternatively, the precipitation can be induced by reducing the temperature of reaction mixture, especially if initial temperature of reaction mixture is elevated. The precipitation can also be induced by reduction of the volume of the solution, preferably under diminished pressure, or by complete evaporation of solvent. Furthermore, the precipitation may be caused by adding an antisolvent.

In one aspect of the invention rasagiline salt is prepared by adding pharmaceutically acceptable acid in natural state or in solution to the solution of racemic N-propargyl-1-aminoindane or rasagiline base in a solvent of medium polarity selected from alcohols, ketones, nitriles, and esters or mixtures thereof, preferably selected from acetone, C₁-C₄ alcohols, acetonitrile and ethyl acetate, most preferably 2-propanol, optionally heating the mixture to obtain a solution, and cooling. The precipitation of salt occurs after long standing the solution at appropriate temperature below 50°C., preferably between -10 to 25°C., after cooling the stirred mixture from heated solution below 50°C., preferably to room temperature or below, both after optional concentration of the solution by partial evaporation of solvents.

In another option the salt is formed by reprecipitation in a suspension of one or both starting components, or by precipitation adding antisolvent preferably selected from ethers and hydrocarbons, most preferably heptane.

In another aspect of the invention rasagiline salts are prepared by adding pharmaceutically acceptable acid in natural state or dissolved to a solution of racemic N-propargyl-1-aminoindane or rasagiline base in lower alcohol, preferably 2-propanol, following by complete or partial evaporation of the solvents.

In another aspect of the present invention rasagiline L-mandelate and rasagiline (+)-camphor-10-sulfonate are prepared by adding L-mandelic acid or (+)-camphor-10-sulfonic acid, respectively, in solid state to a solution of racemic N-propargyl-1-aminoindane or rasagiline base in a solvent of medium polarity, selected from alcohols, ketones, nitriles, and esters, preferably selected from acetone, C₁-C₄ alcohols, acetonitrile and ethyl acetate, most preferably 2-propanol, optionally heating the mixture to obtain a solution and cooling. The precipitation of salt occurs after long standing the solution at appropriate below 50°C., preferably between -20 to 25°C., after cooling the stirred mixture from heated solution below 50°C., preferably to room temperature or below, both after optional concentration of the solution by partial evaporation of solvents. In another option the salt is formed by reprecipitation in a suspension of one or both starting components, or by precipitation adding antisolvent preferably selected from ethers and hydrocarbons; most preferably the antisolvent is heptane.

In another aspect of the present invention rasagiline L-mandelate and rasagiline (+)-camphor-10-sulfonate are prepared by adding L-mandelic acid or (+)-camphor-10-sulfonic acid, respectively, in a solid state to a solution of racemic N-propargyl-1-aminoindane or rasagiline base in lower alcohol preferably 2-propanol following by complete or partial evaporation of the solvents.

In one preferred example rasagiline base and L-mandelic acid in an equimolar ratio or excess to rasagiline base are separately dissolved in a solvent of medium polarity, preferably 2-propanol, to yield two separate solutions. An antisolvent, preferably heptane, is then slowly added to the combined solution to form a precipitate, which is filtered to give crystals of rasagiline L-mandelate.

In another preferred example racemic N-propargyl-1-aminoindane is dissolved in a solvent of medium polarity, preferably 2-propanol, and heated to reflux temperature. To this reaction mixture a solution of L-mandelic acid in a solvent of medium polarity, preferably 2-propanol, in an equimolar ratio or excess to racemic N-propargyl-1-aminoindane is slowly added. The reaction mixture is then cooled to about room temperature and seeded with crystals of rasagiline L-mandelate obtained previously, for example according to procedure as described in the preceding preferred example. Finally, antisolvent, preferably heptane, is slowly added to the obtained dispersion, which is subsequently left to stir at about room temperature for 6 to 24 hours, preferably 12 hours (for example overnight), to give rasagiline L-mandelate.

Rasagiline L-mandelate obtained according to such procedure possesses high enantiomeric purity, preferably over 90%, more preferably over 95% of R(+) enantiomer. Further recrystallization step(s) from solvents, in which rasagiline L-mandelate is significantly more soluble in hot than in cold, preferably in a solvent of medium polarity, selected from alcohols, ketones, nitriles, and esters or water or mixtures thereof, further increases the enantiomeric purity of rasagiline L-mandelate to over 99%, preferably over 99.9% of R(+) enantiomer. Most preferably S(-) enantiomer is not detected by routine chiral HPLC method.

Rasagiline L-mandelate prepared according to such procedure exhibits a powder X-ray diffraction pattern comprising the following characteristic reflection angles 2θ: 5.4±0.2 °, 9.7±0.2 °, 10.8±0.2 °, 17.2±0.2 °, 19.4±0.2 °, 20.5±0.2 °, 21.9±0.2 °, 27.0±0.2 and 29.6:t0.2°.

Rasagiline L-mandelate prepared according to such procedure exhibits melting point at about 107 to 111 °C.

Rasagiline L-mandelate prepared according to such procedure has a D₉₀ particles size of about 300 µm to about 600 µm.

In yet another preferred example rasagiline base and (+)-camphor-10-sulfonic acid in an equimolar ratio or excess to rasagiline base are separately dissolved in a solvent of medium polarity, preferably 2-propanol, to yield two separate solutions. Antisolvent preferably heptane is then slowly added to the combined solution and left to evaporate slowly. After several hours crystals of rasagiline (+)-camphor-10-sulfonate are formed.

In yet another preferred example racemic N-propargyl-1-aminoindane is dissolved in a solvent of medium polarity, preferably 2-propanol and heated to reflux temperature. To this reaction mixture a solution of (+)-camphor-10-sulfonic acid in a solvent of medium polarity, preferably 2-propanol, in an equimolar ratio or excess to racemic N-propargyl-1-aminoindane is slowly added. The reaction mixture is then cooled to about room temperature and seeded with crystals of rasagiline (+)-camphor-10-sulfonate obtained previously, for example according to procedure as described in the preceding preferred example. Finally, antisolvent, preferably heptane, is slowly added to the reaction mixture, which is subsequently left to stir at about room temperature for 6 to 24 hours, preferably 12 hours (for example overnight), to give rasagiline (+)-camphor-10-sulfonate.

Rasagiline (+)-camphor-10-sulfonate obtained according to such procedure possesses high enantiomeric purity, preferably over 90%, more preferably over 95% of R(+) enantiomer. Further recrystallization step(s) from solvents, in which rasagiline (+)-camphor-10-sulfonate is significantly more soluble in hot than in cold, preferably in a solvent of medium polarity, selected from alcohols, ketones, nitriles, and esters or water or mixtures thereof, further increases the enantiomeric purity of rasagiline (+)-camphor-10-sulfonate to over 99%, preferably over 99.9% of R(+) enantiomer. Most preferably S(-) enantiomer is not detected by routine chiral HPLC method.

Rasagiline (+)-camphor-10-sulfonate prepared according to such procedure exhibits a powder X-ray diffraction pattern comprising the following characteristic reflection angles 20: 7.6±0.2 °, 13.1 ±0.2 °, 18.1 ±0.2 °, 19.0±0.2 °, 21.6±0.2 °, 23.1 ±0.2 °, 25.7±0.2 ° and 29.5±0.2 °.

Rasagiline (+)-camphor-10-sulfonate prepared according to such procedure exhibits melting point at about 167 to 170°C.

Rasagiline (+)-camphor-10-sulfonate prepared according to such procedure has a D₉₀ particles size of about 500 µm to about 1000 µm.

In another embodiment, the particle sizes of rasagiline salt of the present invention (e.g. rasagiline L-mandelate and rasagiline (+)-camphor-10-sulfonate), can be further reduced by a mechanical process of reducing the size of particles, which includes any one or more of cutting, chipping, crushing, milling, grinding, micronizing, trituration, or other particle size reduction methods known in the art, to bring the rasagiline salt to the desired particle size range which is suitable for homogeneous distribution of the drug substance in a pharmaceutical dosage form (e.g. tablet).

Another aspect of the present invention is a pharmaceutical composition for administering a therapeutically effective amount of rasagiline salts with pharmaceutically acceptable acid of the present invention, preferably rasagiline L-mandelate and rasagiline (+)-camphor-10-sulfonate, in unit dosage form with one or more pharmaceutically acceptable carriers or other excipients.

A therapeutically effective amount of rasagiline salt of the present invention is amount of salt ranging, when calculated as rasagiline base, from 0.1 to 5 mg, preferably from 0.5 to 2 mg, more preferably from 0.5 to 1 mg.

Pharmaceutically acceptable salts in accordance with present invention can be embodied for example in form of tablets, capsules, pellets, granules and suppositories or their combined forms. Pharmaceutical composition in accordance with present invention can be suitable for immediate release or modified release of rasagiline salts of the present invention. Solid pharmaceutical compositions can be for example coated with aim of increasing palatability or regulating the disintegration or absorption.

Pharmaceutically acceptable excipients may be selected from the group consisting of binders, diluents, disintegrating agents, stabilizing agents, preservatives, lubricants, fragrances, flavoring agents, sweeteners and other excipients known in the field of the pharmaceutical technology. Preferably, carriers and excipients may be selected from the group consisting of lactose, microcrystalline cellulose, cellulose derivatives, (e.g. hydroxypropylcellulose, croscarmellose sodium), polyacrylates, calcium carbonate, starch, maize starch, pregelatinized maize starch, colloidal silicone dioxide, colloidal anhydrous silica, sodium starch glycolate, talc, magnesium stearate, mannitol, polyvinylpyrrolidone, polyethylene glycol and other excipients known in the field of the pharmaceutical technology.

Optionally, the pharmaceutical compositions of the invention may be combination products comprising one or more additional pharmaceutically active components in addition to rasagiline salts.

The pharmaceutical compositions according to the present invention may be prepared by methods known in the field of the pharmaceutical technology.

The further aspect of the present invention is a method for treatment of a monoamine-oxidase type B related disorder in an organism, with a medicament by using an effective amount of rasagiline salts according to the present invention, preferably rasagiline L-mandelate and rasagiline (+)-camphor-10-sulfonate.

In another aspect the present invention is related to use of rasagiline salts according to present invention, preferably rasagiline L-mandelate and rasagiline (+)-camphor-10-sulfonate, for the manufacture of medicament for treatment of a monoamine-oxidase type B related disorder in an organism.

Said monoamine-oxidase type B related disorder is preferably Parkinson's disease, memory disorders, dementia of the Alzheimer type, depression, hyperactive syndrome, a neurotoxic injury, brain ischemia or stroke, head trauma injury or spinal trauma injury, neurotrauma, schizophrenia, attention deficit disorder; multiple sclerosis, nerve damage, an affective illness, symptoms of withdrawal from an addictive substance, amyotrophic lateral sclerosis, multiple system atrophy, restless leg syndrome, glaucoma, and hairloss.

### Experimental Procedures

**Table 1: Solubility of rasagiline salts in water at 25°C and 40°C as measured using Crystal16 ™ from Avantium Technologies.**

| | **25ºC mg/ml** | **40ºC mg/ml** |
|---|---|---|
| | **(H2O)** | **(H2O)** |
| **(+)-camphor-10-sulfonate** | 113 | 175 |
| **L-mandelate** | 70 | 100 |
| **L-tartrate** | <30 | 52 |

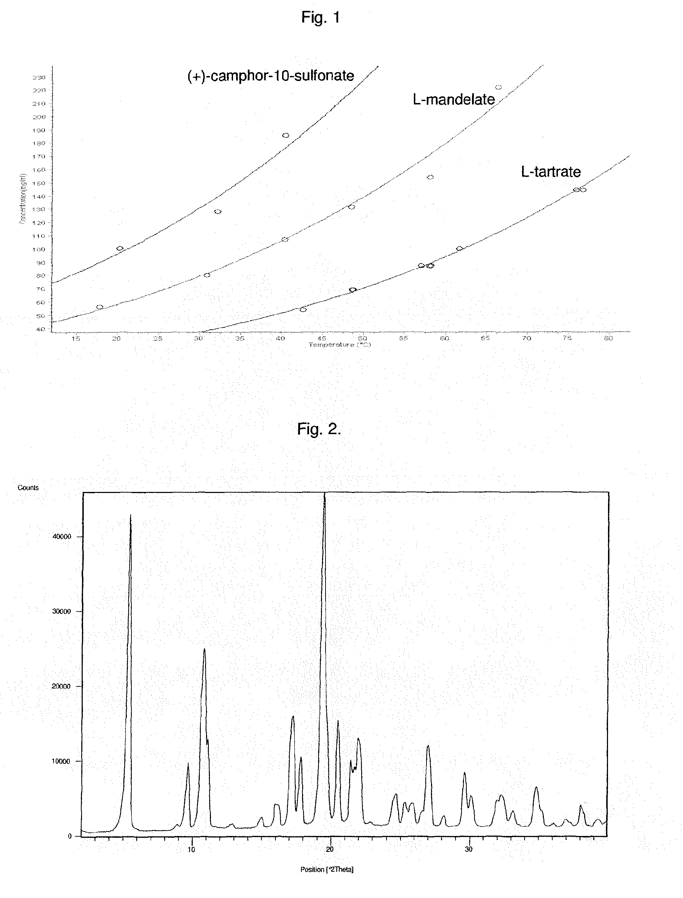

As shown by results illustrated in Table 1 and Fig. 1, both L-mandelate and (+)-camphor-10-sulfonate salts of rasagiline possess markedly increased solubility in water relative to L-tartrate salt.

Crystalline rasagiline free base may be prepared according to the general procedures of WO 08/76348.

Rasagiline L-tartrate was prepared according to the procedure described in international patent application WO 07/61717.

### General procedures of the Examples:

Rasagiline base was dissolved in a suitable solvent. An equimolar solution of acid in a suitable solvent was added to the rasagiline base solution. A suitable antisolvent was then slowly added to the combined solution to form a crystalline precipitate, which was filtered to give rasagiline salt.

Alternatively, racemic N-propargyl-1-aminoindane was dissolved in a suitable solvent and heated to reflux temperature. To this reaction mixture an equimolar solution of acid dissolved in a suitable solvent was slowly added. The reaction mixture was then cooled to room temperature and seeded with crystals of rasagiline salt obtained previously. Finally, a suitable antisolvent was slowly added to the reaction mixture, which was then stirred at room temperature overnight, to give rasagiline salt.

According to this general procedures, appropriate salts were prepared using L-mandelic, (+)-camphor-10-sulfonic, orotic, trans-cinnamic, 1-hydroxy-2-naphthoic, fumaric, benzoic, D-mandelic and (-)-camphor-1 0-sulfonic acid.

Apart from 2-propanol, diethyl ether and water were used to prepare solutions/suspensions of rasagiline base and acid.

### Example 1 (Rasagiline L-mandelate)

Rasagiline base (0.77g) was dissolved in 2-propanol (10 ml) and was combined with solution of L-(+)-mandelic acid (0.68 g) in 2-propanol (10 ml). To this combined solution 20 ml of heptane was slowly added, and thus formed crystalline precipitate was filtered to give rasagiline L-mandelate.

### Example 2 (Rasagiline L-mandelate)

Racemic N-propargyl-1-aminoindane (23.87 g) was dissolved in 2-propanol (100 ml), and solution was heated to reflux temperature. To reaction mixture, a solution of L-mandelic acid (10.6 g) in 2-propanol (20 ml) was slowly added. Reaction mixture was cooled to r.t., seeded with rasagiline L-mandelate from example 1, and then 200 ml of heptane was slowly added and stirred at r.t. overnight to give rasagiline L-mandelate (15.10 g, yield 33%, 94.71 % R(+) enantiomer, melting point: 107-111°C).

### Example 3 (Rasagiline (+)-camphor-1 0-sulfonate)

Rasagiline base (0.50g) was dissolved in 2-propanol (10 ml) and was combined with solution of (+)-camphor-10-sulfonic acid (0.68 g) in 2-propanol (5 ml). To this combined solution 20 ml of heptane was slowly added, and left to evaporate slowly. After several hours crystals of rasagiline (+)-camphor-1 0-sulfonate formed.

### Example 4 (Rasagiline (+)-camphor-10-sulfonate)

Racemic N-propargyl-1-aminoindane (25.65 g) was dissolved in 2-propanol (100 ml), and solution was heated to reflux temperature. To reaction mixture, a solution of (+)-camphor-10-sulfonic acid (17.4 g) in 2-propanol (20 ml) was slowly added. Reaction mixture was cooled to r.t., seeded with rasagiline (+)-camphor-10-sulfonate from example 3, and then 160 ml of heptane was slowly added and stirred at r.t. overnight to give rasagiline (+)-camphor-10-sulfonate (16.14 g, yield 27%, 97.00% R(+) enantiomer, melting point: 167 - 170°C)

### Example 5 (Rasagiline L-mandelate)

Rasagiline L-mandelate from example 2 (1.70 g) was suspended in 2-propanol (10 ml) and heated until clear solution was obtained, then left to cool to room temperature, to give rasagiline L-mandelate with 100% R(+) enantiomer content (S(-) enantiomer not detected; Particle size data: (D₉₀) = 434 µm; (D₅₀) = 117 µm).

### Example 6 (Rasagiline (+)-camphor-10-sulfonate)

Rasagiline (+)-camphor-10-sulfonate from example 4 (1.84 g) was suspended in water (10 ml) and heated until clear solution was obtained, then left to cool to room temperature and seeded with rasagiline (+)-camphor-10-sulfonate from example 4, then left to crystalize to give rasagiline (+)-camphor-10-sulfonate with 100% R(+) enantiomer content (S(-) enantiomer not detected; Particle size data: (D₉₀) = 660 µm; (D₅₀) = 340 µm).

### Example 7 (Rasagiline L-mandelate)

Rasagiline L-mandelate from example 2 (2.98 g) was suspended in water (10 ml) and heated until clear solution was obtained, then left to cool to room temperature, to give rasagiline L-mandelate with 97.9% R(+) enantiomer content.

### Example 8 (Rasagiline (+)-camphor-10-sulfonate)

Rasagiline (+)-camphor-10-sulfonate from example 4 (2.45 g) was suspended in 2-propanol (14 ml) and heated until clear solution was obtained, then left to cool to room temperature to give rasagiline (+)-camphor-10-sulfonate with 99.4% R(+) enantiomer content.

### Example 9 (Rasagiline orotate)

Rasagiline base (1.19 g) was dissolved in 2-propanol (120 ml), then orotic acid (1.08 g) was added, followed by water (6 ml), then a suspension was heated until clear solution was momentarily obtained, then left to cool to r.t and crystallize to give rasagiline orotate.

### Example 10 (Rasagiline cinnamate)

Rasagiline base (0.55 g) was dissolved in diethyl ether (20 ml) and trans-cinnamic acid (0.48g) was added to the solution. The solution was left to slowly evaporate until crystals of rasagiline cinnamate were formed.

### Example 11 (Rasagiline 1-hydroxy-2-naftoate)

Rasagiline base (0.60 g) was dissolved in 2-propanol (15 ml) then 1-hydroxy-2-naftoic acid (0.66 g) was added. To this solution 20 ml of heptane was slowly added and the solution was left to crystallize, to give rasigaline 1-hydroxy-2-naftoate.

### Example 12 (Rasagiline fumarate)

Into solution of rasagiline base (0.54 g) in 2-propanol (15 ml), fumaric acid (0.37 g) was added. To this solution, 20 ml of heptane was slowly added to induce the precipitation of rasagiline fumarate.

### Example 13 (Rasagiline benzoate)

Into solution of rasagiline base (0.66 g) in 2-propanol (10 ml), benzoic acid (0.47 g) was added. To this solution, w0 ml of heptane was slowly added, then left the solution to evaporate slowly until crystals of rasagiline benzoate were formed.

### Example 14 (Rasagiline D-mandelate)

Rasagiline base (0.69g) was dissolved in 2-propanol (20 ml) and was combined with solution of D-(-)-mandelic acid (0.61 g) in 2-propanol (10 ml). To this combined solution 20 ml of heptane was slowly added, then left the solution to evaporate slowly until crystals of rasagiline D-mandelate formed.

### Example 15 (Rasagiline (-)-camphor-10-sulfonate)

Rasagiline base (0.57g) was dissolved in 2-propanol (10 ml) and was combined with solution of (-)-camphor-10-sulfonic acid (0.77 g) in 2-propanol (5 ml). To this combined solution 20 ml of heptane was slowly added, and left to evaporate slowly. After several hours crystals of rasagiline (-)-camphor-10-sulfonate formed.

### Methods of analysis

### Enantiomers ratio determination

A ratio of R(+) and S(-) enantiomer was determined by chiral HPLC method and was expressed by relative area percentage of R(+) enantiomer.

### X-Ray powder diffraction method:

Conditions for obtaining powder X-ray diffraction (XRD) patterns: The powder X-ray diffraction patterns were obtained by methods known in the art using Philips X'Pert PRO diffractometer with X'Celerator detector using CuKα radiation (tube operating at 45 kV and 40 mA) in the Bragg-Brentano (reflection) geometry. Data were recorded from 2 to 40 °-26 in steps of 0.033 °-26 and the measurement time of 50 seconds per step. Variable divergence and antiscatter slits were used to maintain 12 mm of sample length irradiated.

### Differential Scanning Calorimetry:

Conditions for obtaining DSC thermograms: Thermograms were obtained with Mettler Toledo DSC822e differential scanning calorimeter. The sample (1-10 mg) was placed in an unsealed aluminium pan with a hole and heated at 10°C/min in the temperature range from 30 °C to 250 °C.

### Particle size distribution:

Particle size distribution of rasagiline salts was characterized by laser diffraction (Malvern Mastersizer S). Sample cell was small volume sample dispersion cell MS1, presentation was 3NHE, solvent was hexane, stirrer speed was 2000 rpm. The following procedure was used:
Fill the sample cell with hexane, add 100 mg of sodium bis (2-ethylhexyl) sulfosuccinate, align the sizer and measure the background. Add sample into the sample cell until proper obscuration is achieved (10-30%). Analyze when the signal from detectors is stable.

## Claims

1. Rasagiline salt with a pharmaceutically acceptable acid, the pharmaceutically acceptable acid being selected from the group consisting of L-mandelic acid and (+)-camphor-10-sulfonic acid, and hydrates and solvates of said salt.

2. Rasagiline salt according to claim 1, which is rasagiline L-mandelate, or a hydrate or solvate thereof, optionally being in crystalline form or in amorphous form.

3. Rasagiline L-mandelate according to claim 2 being in crystalline form and having a powder X-ray diffraction pattern comprising the following characteristic reflection angles 2θ: 5.4±0.2 °, 9.7±0.2 °, 10.8±0.2 °, 17.2±0.2 °, 19.4±0.2 °, 20.5±0.2 °, 21.9±0.2 °, 27-0±0.2 ° and 29.6±0.2 °.

4. Rasagiline L-mandelate according to any of claims 2 or 3 having a melting point falling in a range of 106 to 118°C, preferably 108 to 114°C.

5. Rasagiline salt according to claim 1, which is rasagiline (+)-camphor-10-sulfonate, or a hydrate or solvate thereof, optionally being in crystalline form or in amorphous form.

6. Rasagiline (+)-camphor-10-sulfonate according to claim 5 being in crystalline form and having a powder X-ray diffraction pattern comprising the following characteristic reflection angles 20: 7.6±0.2 °, 13.1 ±0.2 °, 18.1 ±0.2 °, 19.0±0.2 °, 21.6:t0.2°, 23.1 ±0.2 °, 25.7±0.2 ° and 29.5±0.2 °.

7. Rasagiline (+)-camphor-10-sulfonate according to any of claims 5 or 6 having a melting point falling in a range of 162 to 175°C, preferably 167 to 173°C.

8. A process for the preparation of a rasagiline salt according to any one of claims 1-7 comprising the following steps:
a) providing a mixture comprising racemic N-propargyl-1-aminoindane or rasagiline base, and an organic acid selected from the group consisting of L-mandelic acid and (+)-camphor-10-sulfonic acid;
b) isolating the obtained rasagiline salt.

9. The process according to claim 8, wherein said mixture is provided in 2-propanol as solvent.

10. A pharmaceutical composition comprising a rasagiline salt according to any one of claims 1 to 7.

11. The pharmaceutical composition according to claim 10, wherein said rasagiline salt is rasagiline L-mandelate.

12. The pharmaceutical composition according to claim 10, wherein said rasagiline salt is rasagiline (+)-camphor-10-sulfonate.

13. A pharmaceutical composition according to any one of claims 10 to 12 for use in therapeutic treatment of Parkinson's disease, memory disorders, dementia of the Alzheimer type, depression, hyperactive syndrome, a neurotoxic injury, brain ischemia or stroke, head trauma injury or spinal trauma injury, neurotrauma, schizophrenia, attention deficit disorder; multiple sclerosis, nerve damage, an affective illness, symptoms of withdrawal from an addictive substance, amyotrophic lateral sclerosis, multiple system atrophy, restless leg syndrome, glaucoma, hairloss.
